# DEMANDE DE BREVET EUROPEEN

(11) **EP 0 604 287 A1**
(43) Date de publication de la demande: **29.06.1994**
(21) Numéro de dépôt: 93403085.9
(22) Date de dépôt: 17.12.1993
(51) Int. Cl.: G01N 33/52, G01N 1/28, A61K 7/00

(54) **Méthode et dispositif pour tester la réactivité de cellules à l'égard de produits**

(30) Priorité: 24.12.1992 FR 9215737
(71) Demandeur: LABORATOIRES DE BIOLOGIE VEGETALE YVES ROCHER, F-56200 La Gacilly (FR)
(72) Inventeur: Mougin, Danièle, F-94400 Vitry Sur Seine (FR)
(74) Mandataire: Le Guen, Gérard

(57) **Abrégé**

La présente invention concerne une méthode pour tester la réactivité de cellules vivantes à l'égard d'au moins un produit, comprenant la formation d'une bande sur laquelle sont présentes des cellules, l'application du côté de ladite bande sur lequel sont présentes les cellules sur le fond d'une plaque comportant une série d'ouvertures transversales et l'application du ou desdits produits dans les puits formés par les ouvertures et la bande.

## Description

La présente invention concerne une méthode et un dispositif pour tester la réactivité de cellules à l'égard d'au moins un produit. La présente invention trouve une application notamment dans une méthode pour tester la réactivité des cornéocytes à l'égard de produits cosmétiques.

Avant d'être commercialisés, les produits cosmétiques doivent être testés afin de vérifier leur innocuité. Dans le but d'éviter d'utiliser des animaux pour de tels tests on a mis au point des méthodes ex vivo.

En particulier, Rutherford et al. (British Journal of Dermatology, 91, 503, 1974) ont proposé une méthode pour évaluer l'irritation subie par l'épiderme après traitement par des produits peu irritants et qui consiste à déterminer dans le Stratum Corneum la présence de phosphatase acide (par utilisation de p-nitrophénylphosphate).

Jusqu'à présent, de telles méthodes n'étaient pas utilisables industriellement car elles ne permettaient que de tester un seul produit à la fois.

La présente invention vise à fournir une méthode pour tester la réactivité de cellules et notamment de cornéocytes vis-à-vis de plusieurs produits à la fois.

La présente invention a aussi pour objet une méthode pour tester la réactivité de cellules à l'égard d'au moins un produit, comprenant la réalisation ou l'utilisation d'une bande sur laquelle sont présentes des cellules, l'application du côté de ladite bande sur lequel sont présentes les cellules sur le fond d'une plaque comportant une série d'ouvertures transversales et l'application du ou desdits produits dans les puits formés par les ouvertures et la bande.

Dans une forme avantageuse, la méthode selon l'invention comprend en outre, après l'application de la bande sur le fond de ladite plaque à ouvertures, l'application sur ladite bande d'une plaque qui est pressée contre la bande.

De plus, la plaque qui est pressée contre la bande est avantageusement recouverte d'une feuille de matériau élastique telle qu'une feuille de caoutchouc destinée à être appliquée contre la bande.

La bande utilisée dans la présente invention peut être une bande sur laquelle on a effectué une culture de cellule. Toutefois, dans une forme particulière de réalisation de l'invention, la bande est une bande adhésive et est utilisée pour prélever des cellules par application sur une culture cellulaire, sur un organe humain ou animal et tout particulièrement est utilisée pour prélever des cornéocytes humains par application sur la peau.

La présente invention a également pour objet un dispositif pour la mise en oeuvre de la méthode selon l'invention et qui comprend une plaque comportant une série d'ouvertures transversales, une plaque destinée à être appliquée contre le fond de la plaque munie d'ouvertures et des moyens pour presser les deux plaques l'une contre l'autre.

L'invention sera mieux comprise au vu de la description qui va suivre, faite en se référant aux dessins annexés sur lesquels :
- la Fig. 1 est une vue en perspective illustrant une étape de la méthode selon l'invention;
- la Fig. 2 est une vue en perspective illustrant une étape ultérieure de la méthode selon l'invention.

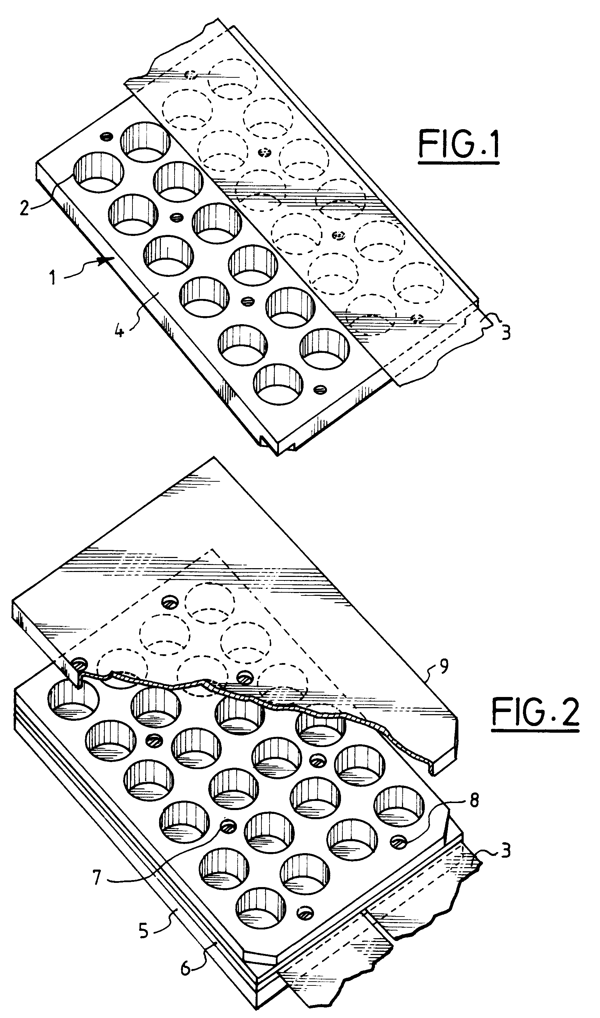

La méthode qui est illustrée sur les Fig. 1 et 2 comprend la mise en oeuvre d'un dispositif comprenant une plaque 1 munie de 4 rangées d'ouvertures transversales 2 à section circulaire.

Sur la plaque 1 on applique une bande adhésive telle que 3 qui vient recouvrir sur toute leur longueur deux rangées d'ouvertures 2. La bande 3 qui est appliquée sur la plaque 1 a été au préalable appliquée sur le dos d'un volontaire et retirée lentement, afin de prélever des cornéocytes.

Sur le fond 4 de la plaque 1, on applique une plaque continue 5 recouverte sur sa face supérieure d'une feuille de caoutchouc 6. L'ensemble est assemblé par des vis 7 introduites dans des percements 8 de la plaque 1 et coopérant avec des filetages noyés dans la masse de la plaque 4.

Le serrage des vis 7 assure l'étanchéité complète du dispositif. Un couvercle 9 permet de mettre les cellules prélevées à l'abri de la poussière.

Dans les différents puits ainsi formés par les parois des ouvertures 2 et la bande adhésive 3, il est possible d'introduire différents produits cosmétiques ou même le même produit cosmétique pour tester la réactivité des cellules, par exemple des cornéocytes vis-à-vis de ce ou de ces produits cosmétiques.

A cet effet, avec des puits de 16 mm de diamètre, on peut introduire 1 ml de produit par puits.

On met à incuber les plaques à la température souhaitée (par exemple 37°C) pendant le temps souhaité (par exemple 2 heures).

A titre d'exemple, on a ajouté après incubation 1 ml d'une solution de p-nitrophénylphosphate en milieu citrate et on a laissé incubé. Puis on a stoppé la réaction par addition de 1 ml de soude 0,1 M dans chaque puits.

Le contenu de chaque puits est alors transvasé dans des cuves pour une détermination colorimétrique à 400 nm de l'activité phosphatase acide des cornéocytes.

## Revendications

1. Méthode pour tester la réactivité de cellules vivantes à l'égard d'au moins un produit, comprenant la formation d'une bande sur laquelle sont présentes des cellules, l'application du côté de ladite bande sur lequel sont présentes les cellules sur le fond d'une plaque comportant une série d'ouvertures transversales et l'application du ou desdits produits dans les puits formés par les ouvertures et la bande.

2. Méthode selon la revendication 1, comprenant en outre, après l'application de la bande sur le fond de ladite plaque à ouvertures, l'application sur ladite bande d'une plaque qui est pressée contre la bande.

3. Méthode selon la revendication 1 ou 2, dans laquelle la plaque qui est pressée contre la bande est recouverte d'une feuille de matériau élastique.

4. Méthode selon l'une des revendications 1 à 3, caractérisée en ce que la bande est formé par prélèvement des cellules par application sur une culture cellulaire, sur un organe humain ou animal d'une bande adhésive.

5. Méthode selon la revendication 4 pour tester la réactivité de cornéocytes humains à l'égard de produits cosmétiques, comprenant le prélèvement de cornéocytes par application sur la peau d'une bande adhésive, l'application de ladite bande adhésive portant les cornéocytes prélevés sur le fond d'une plaque comportant une série d'ouvertures transversales et l'application desdits produits cosmétiques dans les puits formés par les ouvertures et la bande.

6. Dispositif pour la mise en oeuvre d'une méthode selon la revendication 1, comprenant une plaque (1) comportant une série d'ouvertures transversales (2), une plaque (5) destinée à être appliquée contre le fond (4) de la plaque (1) munie d'ouvertures (2) et des moyens (7)pour presser les deux plaques l'une contre l'autre.

7. Dispositif selon la revendication 6, dans lequel la plaque (5) destinée à être pressée contre la plaque (1) munie d'ouvertures (2) est recouverte d'une feuille de matériau élastique (6).
